# EUROPEAN PATENT SPECIFICATION

(11) **EP 4 183 324 B1**
(45) Date of publication and mention of the grant of the patent: **30.09.2026**
(21) Application number: 21842390.3
(22) Date of filing: 07.07.2021
(51) Int. Cl.: A61B 5/00

(54) **SCAN HEAD ASSEMBLY**
ABTASTKOPFANORDNUNG
ENSEMBLE TÊTE DE BALAYAGE

(30) Priority: 14.07.2020 CN 202010676567
(43) Date of publication of application: 24.05.2023
(73) Proprietor: Shining 3D Tech Co., Ltd., Hangzhou, Zhejiang 311258 (CN)
(72) Inventor: FENG, Hailong, Hangzhou, Zhejiang 311258 (CN); ZHANG, Wei, Hangzhou, Zhejiang 311258 (CN)
(74) Representative: Novagraaf Group
(86) International application number: PCT/CN2021/105047
(87) International publication number: WO 2022/012392

(56) References cited:
- EP-A1- 3 597 146
- WO-A2-2018/035026
- CN-A- 109 770 837
- CN-A- 110 584 578
- CN-U- 208 426 093
- CN-U- 212 326 382
- US-A1- 2010 322 282
- US-A1- 2020 170 497
- US-B1- 6 789 936

## Description

### Technical Field

The invention relates to the technical field of scanning equipment, and particularly relates to a scanner head assembly of an intraoral scanner.

### Background

When an intraoral scanner is used to collect data of a patient, a conventional scanner head is preferred in order to ensure the optimal scanning efficiency. For a patient with particularly small space between the lips and teeth, it is necessary to use a flatter scanner head. For a patient who cannot open his mouth properly, it is necessary to use scanner heads of different angles.

When a differential scanner head is used to collect data, due to differences in area cropping size, image transposition, image correction and other factors, a software backend needs to call different algorithms and equations according to the state. The scanner head assembly in the prior art requires manual selection by an operator, which is inconvenient to operate and prone to wrong selection.

A Dental Measuring Device, a Tip End Portion of Dental Measuring Device, and a Dental Measuring System are disclosed in EP3597146A1.

### Summary

A main purpose of the invention is to provide a scanner head assembly to solve the problem that it is not convenient for the scanner head assembly in the art known to inventors to switch between scanning states when using different scanner heads.

In order to achieve the above purpose, some embodiments of the invention provide a scanner head assembly of an intra-oral scanner, comprising: a plurality of types scanner heads, wherein each type of the scanner heads comprises a window and a scanner head body having a cylinder with at least one switch rib piece arranged on its inner wall, different types of the scanner heads have different windows; a scanner host, wherein the scanner host comprises a base section and an insertion head section forming an integral structure with the base section and configured to be nested in the cylinder of the scanner head, and the insertion head section is provided with at least one switch configured to be fitted with the at least one switch rib piece of the scanner head; anda computer which calls parameters according to the state of the at least one switch of the insertion head section, including cropping parameters of the windows corresponding to the scanner head.In some embodiments, each switch rib piece is a switch rib protruding from an inner surface of the scanner head body. The switch rib extends from a side close to the scanner host to a side away from the scanner host, and the side of the switch rib close to the scanner host has a guide slope and a pressing plane. The guide slope gradually heightens inward from the side close to the scanner host to the side away from the scanner host, and the side of the guide slope away from the scanner host is connected to the pressing plane and has a same height as the pressing plane.

In some embodiments, the number of the at least one switch rib pieces is less than or equal to the number of the at least one switches.

In some embodiments, there are two switches. No switch rib piece is arranged in the scanner head body, or one switch rib piece is arranged in the scanner head body, or two switch rib pieces are arranged in the scanner head body.

In some embodiments, the scanner head assembly further includes first limiting portions, and the first limiting portions are arranged on an inner wall of the cylinder and fitted with upper and lower surfaces of the insertion head section.

In some embodiments, the first limiting portions are first limiting ribs, and the first limiting ribs extend along a movement direction of the insertion head section.

In some embodiments, the scanner head assembly further includes second limiting portions, and the second limiting portions are fitted with two side surfaces of the insertion head section.

In some embodiments, the second limiting portions are second limiting ribs, the second limiting ribs are arranged on the inner wall of the cylinder and located on two sides of each first limiting portion, and a height of the second limiting ribs is greater than a height of the each first limiting portion.

In some embodiments, the scanner host is fitted and connected with the scanner head through a snap-fit structure.

In some embodiments, the windows of different types of the scanner heads have different sizes, and/or the windows of different types of the scanner heads have different directions.

According to the technical solution of some embodiments in the invention, when the scanner head assembly is used for scanning, different scanner heads are used according to different use scenarios so as to achieve the best scanning state. The insertion head section of the scanner host is nested in the cylinder of the scanner head, and the switch is fitted with the scanner head to realize control of the scanner host. With the control of the scanner host realized by the scanner head fitted with the switch, there is no need of manual selection for an operator, which greatly facilitates the use for the operator. The technical solution of the invention effectively solves the problem that it is not convenient for the scanner head assembly in the art known to inventors to switch between scanning states when using different scanner heads.

### Brief Description of the Drawings

The accompanying drawings, which constitute a part of the invention, are used to provide a further understanding of the invention, and the exemplary embodiments of the invention and the description thereof are used to explain the invention and do not constitute improper limitations to the invention. In the accompanying drawings:
FIG. 1 shows a schematic structural view of an embodiment of a scanner head assembly according to the invention;
FIG. 2 shows a schematic sectional view of a scanner host of the scanner head assembly in FIG. 1;
FIG. 3 shows a schematic three-dimensional structural view of the scanner host of the scanner head assembly in FIG. 1;
FIG. 4 shows a schematic three-dimensional structural view of the scanner host in FIG. 3 from another angle;
FIG. 5 shows a schematic three-dimensional structural view of a scanner head of the scanner head assembly in FIG. 1; and
FIG. 6 shows a schematic three-dimensional structural view of a metal heat transfer member of the scanner head assembly in FIG. 1.

The accompanying drawings include the following reference signs:
10, scanner host; 11, base section; 12, insertion head section; 121, switch; 122, two side surfaces; 20, scanner head; 21, scanner head body; 22, switch rib piece; 23, first limiting portion; 24, second limiting portion; 25, snap-fit structure; 251, transverse rib; 252, vertical rib; 253, limiting boss; 26, metal heat transfer member; 261, metal plate body; 262, mirror mounting rack; 263, limiting protruding sheet; 264, extension cantilever; 100, reflecting mirror.

### Detailed Description of the Embodiments

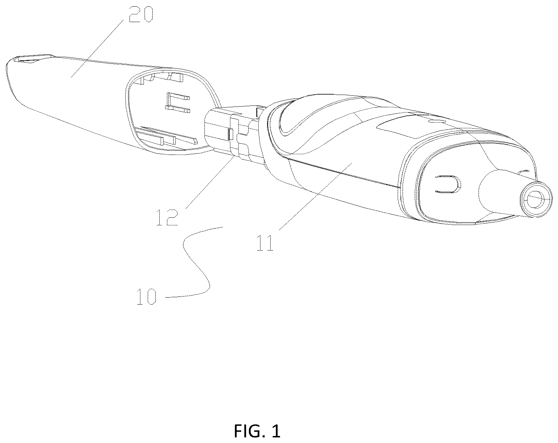

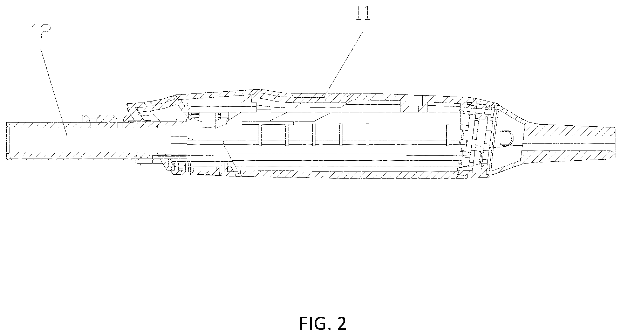

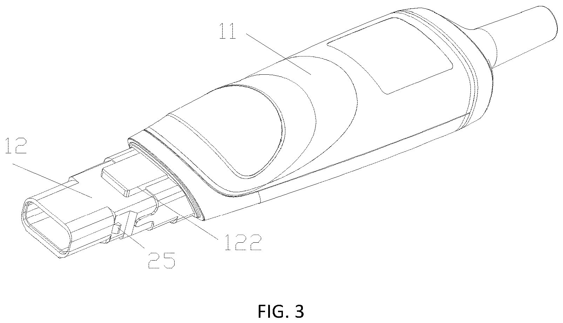

As shown in FIG. 1 to FIG. 6, a scanner head assembly of the embodiment includes: a scanner host 10 and a scanner head 20. The scanner host 10 includes a base section 11 and an insertion head section 12 forming an integral structure with the base section 11, and the insertion head section 12 is provided with at least one switch 121. The scanner head 20 includes a scanner head body 21. The scanner head body 21 has a cylinder. The insertion head section 12 is nested in the cylinder of the scanner head 20, and the at least one switch 121 is fitted with the scanner head 20 to realize control of the scanner host 10.

According to the technical solution of the embodiment, when the scanner head assembly is used for scanning, different scanner heads 20 can be used according to different use scenarios so as to achieve the best scanning state. The insertion head section 12 of the scanner host 10 is nested in the cylinder of the scanner head 20, and the switch 121 is fitted with the scanner head 20 to realize control of the scanner host 10. With the control of the scanner host 10 realized by the scanner head 20 fitted with the switch 121, there is no need of manual selection for an operator, which greatly facilitates the use for the operator. The technical solution of the embodiment effectively solves the problem that it is not convenient for the scanner head assembly in the art known to inventors to switch between scanning states when using different scanner heads.

It should be noted that a scanner of the invention is an intraoral three-dimensional scanner. The scanner host 10 includes a projection module and a camera module. The projection module is configured to project patterns. The camera module collects the patterns and reproduces the patterns at a terminal. The terminal includes a mobile terminal, such as a mobile phone, etc., or the terminal includes a computer, etc. The integral structure of the base section 11 and the insertion head section 12 is an integral structure formed by assembling the base section 11 and the insertion head section 12. A first end of the scanner head 20 has an opening of the cylinder, and the insertion head section 12 is inserted from the opening of the scanner head 20. A second end of the scanner head 20 has a scanning port (window). For example, the scanning port is arranged on a side wall of the scanner head 20, and the scanner head 20 has a reflecting surface therein. In some embodiments, the reflecting surface is a surface of a plane mirror. Light projected by the scanner host 10 is reflected by the reflecting surface to a target object. The light is reflected by a surface of the target object to the reflecting surface, reflected by the reflecting surface, and received by the scanner host 10. The switch 121 may be a contact switch or an inductive switch. The control of the scanner host 10 realized by the scanner head 20 fitted with the switch 121 is mainly embodied in the switching of modes. The computer recognizes the scanner head in use according to the state of the contact switch. The computer calls corresponding parameters, which mainly involves cropping parameters of the window corresponding to the scanner head.

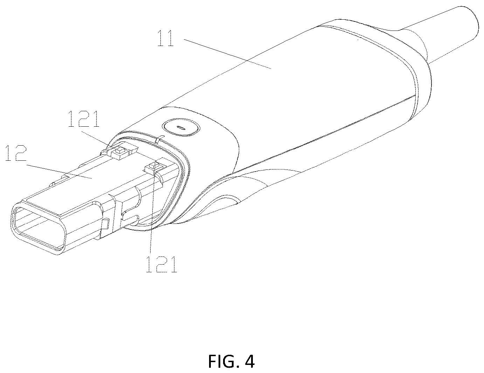

As shown in FIG. 4, in the technical solution of the embodiment, there is one or a plurality of switches 121. When there is one switch, the processing cost is lower. When there is a plurality of switches 121, the scanner head 20 can be fitted with the switches 121 in many forms, which can control many scenarios of the scanner host 10. With n representing the number of the switches 121, 2n types of scanner heads are realized. For example, if the number of the switches 121 is two, the switches can be fitted with the scanner head 20 in four forms: 1. the scanner head 20 is in contact with neither of the switches 121; 2. the scanner head 20 is in contact with one of the switches 121, in two forms; and 3. the scanner head 20 is in contact with the two switches 121.

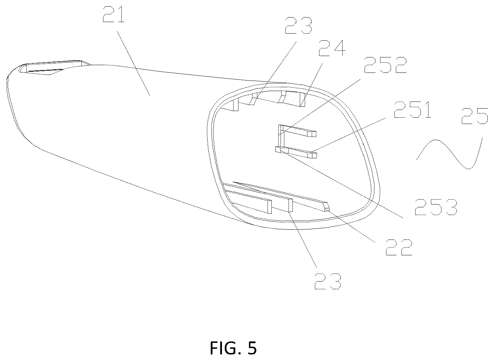

As shown in FIG. 5, in the technical solution of the embodiment, the scanner head 20 further includes a switch rib piece 22. The switch rib piece 22 is arranged on an inner wall of the cylinder, and the switch rib piece 22 is fitted with the switch 121 to realize control of the scanner host 10. The structure above is compact, and the processing cost is lower. The switch rib piece 22 is arranged according to actual conditions, and the switch rib piece 22 may have a variety of shapes. The switch rib piece 22 has a guide slope. When the scanner head is inserted into the scanner host, the guide slope moves relative to the switch and presses the switch at the same time, which thereby avoids damaging the switch 121. Specifically, the switch rib piece 22 is a switch rib protruding from an inner surface of the scanner head body 21. The switch rib extends from a side close to the scanner host 10 to a side away from the scanner host 10, and the side of the switch rib close to the scanner host 10 has a guide slope and a pressing plane. The guide slope gradually heightens inward from a side close to the scanner host 10 to a side away from the scanner host 10, and the side of the guide slope away from the scanner host 10 is connected to the pressing plane and has a same height as the pressing plane. With the above structure, the switch rib piece 22 is fitted with the switch 121 gently, which prevents a problem that the switch 121 is damaged due to the switch rib piece 22 pressing the switch 121 hard.

As shown in FIG. 1 to FIG. 5, in the technical solution of the embodiment, when there are a plurality of switches 121, there may be one switch rib piece 22, or there may be a plurality of switch rib pieces 22 less than or equal to the number of switches 121. With the above structure, the scanner head 20 can be fitted with the scanner host 10 in many forms. The number of the switch rib pieces 22 may also be zero, which is also a control mode. In some embodiments, the number of the switches 121 is two, and there are four structural forms for the switch rib piece 22 in the scanner head 20: no switch rib piece 22 is arranged in the scanner head body 21, or one switch rib piece 22 is arranged in the scanner head body 21, or two switch rib pieces 22 are arranged in the scanner head body 21. The switch rib piece 22 is a strip-shaped structure protruding from an inner wall of the scanner head body 21.

As shown in FIG. 5, in the technical solution of the embodiment, the scanner head assembly further includes first limiting portions 23, and the first limiting portions 23 are arranged on an inner wall of the cylinder and fitted with upper and lower surfaces of the insertion head section 12. With the above structure, the scanner head 20 can limit the scanner host 10 in the up-down direction. In some embodiments, the first limiting portions 23 are first limiting ribs, and the first limiting ribs extend along a movement direction of the insertion head section 12. The structure of the first limiting ribs reduces the processing cost. For example, compared with the plane, the processed surface of the first limiting rib has a greatly reduced processing area. With the first limiting ribs, the amount of the material used is also reduced. The structure of the first limiting ribs is able to buffer the deformation of the scanner host 10. It should be noted that the limiting rib has a guide surface. With the arrangement of the guide surface, the scanner host 10 is easily fitted with the scanner head 20.

As shown in FIG. 1 to FIG. 5, in the technical solution of the embodiment, the scanner head assembly further includes second limiting portions 24, and the second limiting portions 24 are fitted with two side surfaces of the insertion head section 12. The second limiting portions 24 limit the insertion head section 12 in the left-right direction. In some embodiments, the second limiting portions 24 are second limiting ribs, the second limiting ribs are arranged on the inner wall of the cylinder and located on two sides of the first limiting portion 23, and the second limiting ribs have a height greater than that of the first limiting portion 23. The above structure is lower in processing cost. The number of the second limiting ribs is two. The two second limiting ribs have draft angles. In this way, the deeper the insertion of the scanner head 20 into the scanner host 10 is, the tighter the cooperation between the scanner head 20 and the scanner host 10 is. Specifically, an upper part of the insertion head section 12 has a cuboid-shaped bump which is close to the base section 11, and the two side surfaces 122 of the bump are fitted with the second limiting portions 24. In this way, it is only necessary to finish the two side surfaces 122 of the bump. The above structure is compact and lower in processing cost. It should be noted that as shown in FIG. 5, the second limiting ribs are located on the two sides of the upper inner wall of the scanner head 20, and the bump is only located at the upper part of the scanner host 10, so there is no need to provide the second limiting ribs at the lower inner wall of the scanner head 20.

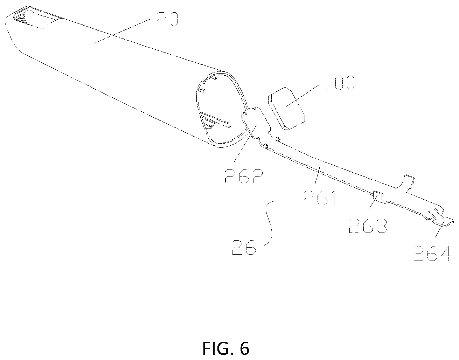

As shown in FIG. 1 and FIG. 6, in the technical solution of the embodiment, the scanner host 10 is fitted and connected with the scanner head 20 through a snap-fit structure 25. The above structure is convenient to operate and compact. The inner wall of the scanner head body has clamping positions, and the two sides of the insertion head section 12 have clamping blocks. A side of each clamping block facing the scanner head 20 has an inclined surface, so that the scanner head 20 can be easily snapped into the insertion head section 12 by the snap-fit structure. There are two clamping positions respectively arranged on the inner wall on two sides of the scanner head body 21. Each of the clamping positions includes two transverse ribs 251 and one vertical rib 252. The two transverse ribs 251 extend along an insertion direction of the scanner host 10. The vertical rib 252 extends along a direction perpendicular to the scanner host 10. The two transverse ribs 251 are arranged in parallel at an interval. The vertical rib 252 is arranged at an end of the transverse ribs away from the scanner host 10. Two ends of the vertical rib 252 are respectively connected to the end of each of the two transverse ribs 251. The clamping block forms a snap fit with the vertical rib 252 through elastic deformation. Similarly, when the scanner host 10 is separated from the scanner head 20, the clamping block is separated from the vertical rib 252 through elastic deformation. In some embodiment, the scanner head assembly further includes a metal heat transfer member 26. The metal heat transfer member 26 includes a metal plate body 261, a mirror mounting rack 262, two limiting protruding plates 263 and an extension cantilever 264. A reflecting mirror 100 is mounted on the mirror mounting rack 262. The mirror mounting rack 262 is located at an end of the metal plate body 261 away from the scanner host 10. The extension cantilever 264 is located at an end of the metal plate body 261 close to the scanner host 10. The two limiting protruding plates 263 are located on two sides of the metal plate body 261. Surfaces of the two transverse ribs away from their fixed ends are depressed. Each of the clamping positions further includes a limiting boss 253. The limiting boss 253 is located at an end of the lower transverse rib away from the scanner host 10. The limiting boss 253 has a height greater than that of the vertical rib. The limiting protruding plates of the metal heat transfer member are located on a side of the limiting boss 253 away from the scanner host 10, which thereby can limit the metal heat transfer member. The arrangement of the metal heat transfer member is able to avoid fogging and condensation of water droplets on the mirror.

In the technical solution of the embodiment, the scanner head 20 includes a plurality of types of scanner heads, and each type of the scanner heads 20 has a window. Different types of the scanner heads 20 have different windows, and different types of the scanner heads 20 correspond to different on/off states of the scanner host 10. The windows of different types of the scanner heads 20 have different sizes, and/or the windows of different types of the scanner heads 20 have different directions. For example, the first type of scanner head has one switch rib piece 22, and has a larger window facing upward. The second type of scanner head has two switch rib pieces 22, and has a larger window facing the left side of the scanner. The third type of scanner head has one switch rib piece 22, which is in a different position from the switch rib piece 22 of the first type of scanner head and corresponds to a different switch from the scanner host. The third type of scanner head has a smaller size than the first type of scanner head, and the third type of scanner head has a smaller window than the first type of scanner head. The scanner assembly of the embodiment may include one type of scanner head or a plurality of types of scanner heads.

As shown in FIG. 5, in the technical solution of the embodiment, the scanner head 20 is made of plastic. Thus, when the scanner head 20 is disinfected by high temperature and high pressure, or by being wiped or soaked with a chemical reagent, the scanner head 20 is not prone to chemical corrosion damage. It should be noted that in the technical solution of the embodiment, the reflecting surface in the scanner head 20 is made of glass.

## Claims

1. A scanner head assembly of an intra-oral scanner, comprising:
a plurality of types of scanner heads (20), wherein each type of the scanner heads (20) comprises a window and a scanner head body (21) having a cylinder with at least one switch rib piece (22) arranged on its inner wall, different types of the scanner heads (20) have different windows;
a scanner host (10), wherein the scanner host (10) comprises a base section (11) and an insertion head section (12) forming an integral structure with the base section (11) and configured to be nested in the cylinder of the scanner head (20), and the insertion head section (12) is provided with at least one switch (121) configured to be fitted with the at least one switch rib piece (22) of the scanner head (20); and
a computer which calls parameters according to the state of the at least one switch of the insertion head section, including cropping parameters of the window corresponding to the scanner head.

2. The scanner head assembly according to claim 1, **characterized in that** the each switch rib piece (22) is a switch rib protruding from an inner surface of the scanner head body (21), the switch rib extends from a side close to the scanner host (10) to a side away from the scanner host (10), the side of the switch rib close to the scanner host (10) has a guide slope and a pressing plane, the guide slope gradually heightens inward from the side close to the scanner host (10) to the side away from the scanner host (10), and the side of the guide slope away from the scanner host (10) is connected to the pressing plane and has a same height as the pressing plane.

3. The scanner head assembly according to claim 1, **characterized in that** the number of the at least one switch rib piece (22) is less than or equal to that of the at least one switch (121).

4. The scanner head assembly according to claim 3, wherein there are two switches (121), and one switch rib piece (22) is arranged in the scanner head body (21), or two switch rib pieces (22) are arranged in the scanner head body (21).

5. The scanner head assembly according to claim 1, **characterized in that** the scanner head assembly further comprises first limiting portions (23), and the first limiting portions (23) are arranged on an inner wall of the cylinder and fitted with upper and lower surfaces of the insertion head section (12).

6. The scanner head assembly according to claim 5, **characterized in that** the first limiting portions (23) are first limiting ribs, and the first limiting ribs extend along a movement direction of the insertion head section (12).

7. The scanner head assembly according to claim 5, **characterized in that** the scanner head assembly further comprises second limiting portions (24), and the second limiting portions (24) are fitted with two side surfaces (122) of the insertion head section (12).

8. The scanner head assembly according to claim 7, **characterized in that** the second limiting portions (24) are second limiting ribs, the second limiting ribs are arranged on the inner wall of the cylinder and located on two sides of each first limiting portion (23), and a height of the second limiting ribs is greater than a height of the each first limiting portion (23).

9. The scanner head assembly according to claim 1, wherein the scanner host (10) is fitted and connected with the scanner head (20) through a snap-fit structure (25).

10. The scanner head assembly according to claim 1, **characterized in that** the windows of different types of the scanner heads (20) have different sizes, and/or the windows of different types of the scanner heads (20) have different directions.

## Patentansprüche

1. Scannerkopfanordnung eines intraoralen Scanners, umfassend:
eine Vielzahl von Arten von Scannerköpfen (20), wobei jede Art der Scannerköpfe (20) ein Fenster und einen Scannerkopfkörper (21) umfasst, der einen Zylinder mit mindestens einem Schaltrippenstück (22) aufweist, das an seiner Innenwand angeordnet ist, wobei unterschiedliche Arten der Scannerköpfe (20) unterschiedliche Fenster aufweisen;
einen Scannerhost (10), wobei der Scannerhost (10) einen Basisbereich (11) und einen Einführkopfbereich (12) umfasst, der eine integrale Struktur mit dem Basisbereich (11) ausbildet und konfiguriert ist, um in dem Zylinder des Scannerkopfes (20) verschachtelt zu sein, und der Einführkopfbereich (12) mit mindestens einem Schalter (121) versehen ist, der konfiguriert ist, um mit dem mindestens einen Schalterrippenstück (22) des Scannerkopfes (20) zusammenzupassen; und
einen Computer, der Parameter gemäß dem Zustand des mindestens einen Schalters des Einführkopfbereichs abruft, die Zuschneideparameter des Fensters einschließen, das dem Scannerkopf entspricht.

2. Scannerkopfanordnung nach Anspruch 1,
**dadurch gekennzeichnet, dass** das jeweilige Schalterrippenstück (22) eine Schalterrippe ist, die von einer Innenoberfläche des Scannerkopfkörpers (21) vorsteht, wobei sich die Schalterrippe von einer dem Scannerhost (10) nahen Seite zu einer dem Scannerhost (10) fernen Seite erstreckt, die dem Scannerhost (10) nahe Seite der Schalterrippe eine Führungsschräge und eine Druckebene aufweist, sich die Führungsschräge von der dem Scannerhost (10) nahen Seite zu der dem Scannerhost (10) fernen Seite nach innen allmählich erhöht und die dem Scannerhost (10) ferne Seite der Führungsschräge mit der Druckebene verbunden ist und eine gleiche Höhe wie die Druckebene aufweist.

3. Scannerkopfanordnung nach Anspruch 1, **dadurch gekennzeichnet, dass** die Anzahl des mindestens einen Schalterrippenstücks (22) kleiner oder gleich der des mindestens einen Schalters (121) ist.

4. Scannerkopfanordnung nach Anspruch 3, wobei zwei Schalter (121) vorhanden sind und ein Schalterrippenstück (22) in dem Scannerkopfkörper (21) angeordnet ist oder zwei Schalterrippenstücke (22) in dem Scannerkopfkörper (21) angeordnet sind.

5. Scannerkopfanordnung nach Anspruch 1,
**dadurch gekennzeichnet, dass** die Scannerkopfanordnung ferner erste Begrenzungsabschnitte (23) umfasst und die ersten Begrenzungsabschnitte (23) an einer Innenwand des Zylinders angeordnet sind und mit oberen und unteren Oberflächen des Einführkopfbereichs (12) zusammenpassen.

6. Scannerkopfanordnung nach Anspruch 5,
**dadurch gekennzeichnet, dass** die ersten Begrenzungsabschnitte (23) erste Begrenzungsrippen sind und sich die ersten Begrenzungsrippen entlang einer Bewegungsrichtung des Einführkopfbereichs (12) erstrecken.

7. Scannerkopfanordnung nach Anspruch 5,
**dadurch gekennzeichnet, dass** die Scannerkopfanordnung ferner zweite Begrenzungsabschnitte (24) umfasst und die zweiten Begrenzungsabschnitte (24) mit zwei Seitenoberflächen (122) des Einführkopfbereichs (12) zusammenpassen.

8. Scannerkopfanordnung nach Anspruch 7, **dadurch gekennzeichnet, dass** die zweiten Begrenzungsabschnitte (24) zweite Begrenzungsrippen sind, die zweiten Begrenzungsrippen an der Innenwand des Zylinders angeordnet sind und sich auf zwei Seiten jedes ersten Begrenzungsabschnitts (23) befinden und eine Höhe der zweiten Begrenzungsrippen größer als eine Höhe jedes ersten Begrenzungsabschnitts (23) ist.

9. Scannerkopfanordnung nach Anspruch 1, wobei der Scannerhost (10) mit dem Scannerkopf (20) durch eine Schnappverbindungsstruktur (25) zusammenpasst und verbunden ist.

10. Scannerkopfanordnung nach Anspruch 1,
**dadurch gekennzeichnet, dass** die Fenster unterschiedlicher Arten der Scannerköpfe (20) unterschiedliche Größen aufweisen und/oder die Fenster unterschiedlicher Arten der Scannerköpfe (20) unterschiedliche Richtungen aufweisen.

## Revendications

1. Ensemble tête de balayaged'un dispositif de balayage intra-oral, comprenant :
une pluralité de types de têtes de balayage (20), dans lequel chaque type de têtes de balayage (20) comprend une fenêtre et un corps de tête de balayage (21) ayant un cylindre avec au moins une pièce de nervure de commutateur (22) agencée sur sa paroi interne, différents types de têtes de balayage (20) ont des fenêtres différentes ;
un hôte de balayage (10), dans lequel l'hôte de balayage (10) comprend une section de base (11) et une section de tête d'insertion (12) formant une structure intégrale avec la section de base (11) et conçue pour s'emboîter dans le cylindre de la tête de balayage (20), et la section de tête d'insertion (12) est pourvue d'au moins un commutateur (121) configuré pour être équipé de l'au moins une pièce de nervure de commutateur (22) de la tête de balayage (20) ; et
un ordinateur qui appelle des paramètres conformément à l'état de l'au moins un commutateur de la section de tête d'insertion, y compris des paramètres de recadrage de la fenêtre correspondant à la tête de balayage.

2. Ensemble tête de balayage selon la revendication 1,
**caractérisé en ce que** chaque pièce de nervure de commutateur (22) est une nervure de commutateur faisant saillie à partir d'une surface interne du corps de tête de balayage (21), la nervure de commutateur s'étend à partir d'un côté proche de l'hôte de balayage (10) vers un côté éloigné de l'hôte de balayage (10), le côté de la nervure de commutateur proche de l'hôte de balayage (10) a une pente de guidage et un plan de pressage, la pente de guidage s'accentue progressivement vers l'intérieur du côté proche de l'hôte de balayage (10) au côté opposé à l'hôte de balayage (10), et le côté de la pente de guidage opposé à l'hôte de balayage (10) est relié au plan de pressage et a une même hauteur que le plan de pressage.

3. Ensemble tête de balayage selon la revendication 1,
**caractérisé en ce que** le nombre de l'au moins une pièce de nervure de commutateur (22) est inférieur ou égal à celui de l'au moins un commutateur (121).

4. Ensemble tête de balayage selon la revendication 3, dans lequel il y a deux commutateurs (121), et une pièce de nervure de commutateur (22) est agencée dans le corps de tête de balayage (21), ou deux pièces de nervure de commutateur (22) sont agencées dans le corps de tête de balayage (21).

5. Ensemble tête de balayage selon la revendication 1,
**caractérisé en ce que** l'ensemble tête de balayage comprend en outre des premières parties de limitation (23), et les premières parties de limitation (23) sont agencées sur une paroi interne du cylindre et équipées de surfaces supérieure et inférieure de la section de tête d'insertion (12).

6. Ensemble tête de balayage selon la revendication 5,
**caractérisé en ce que** les premières parties de limitation (23) sont des premières nervures de limitation, et les premières nervures de limitation s'étendent le long d'un sens de mouvement de la section de tête d'insertion (12).

7. Ensemble tête de balayage selon la revendication 5,
**caractérisé en ce que** l'ensemble tête de balayage comprend en outre des secondes parties de limitation (24), et les secondes parties de limitation (24) sont équipées de deux surfaces latérales (122) de la section de tête d'insertion (12).

8. Ensemble tête de balayage selon la revendication 7,
**caractérisé en ce que** les secondes parties de limitation (24) sont des secondes nervures de limitation, les secondes nervures de limitation sont agencées sur la paroi interne du cylindre et situées sur deux côtés de chaque première partie de limitation (23), et une hauteur des secondes nervures de limitation est supérieure à une hauteur de chaque première partie de limitation (23).

9. Ensemble tête de balayage selon la revendication 1, dans lequel l'hôte de balayage (10) est équipé de et relié à la tête de balayage (20) par le biais d'une structure à encliquetage (25).

10. Ensemble tête de balayage selon la revendication 1,
**caractérisé en ce que** les fenêtres de différents types de têtes de balayage (20) ont des tailles différentes, et/ou les fenêtres de différents types de têtes de balayage (20) ont des sens différents.
